(19) Europäisches Patentamt
European Patent Office
Office européen des brevets

(11) **EP 0 990 211 B1**

(12) **EUROPÄISCHE PATENTSCHRIFT**

(45) Veröffentlichungstag und Bekanntmachung des Hinweises auf die Patenterteilung:
**29.08.2001 Patentblatt 2001/35**

(21) Anmeldenummer: **98932154.2**

(22) Anmeldetag: **15.06.1998**

(51) Int Cl.⁷: $G06F\ 17/00$

(86) Internationale Anmeldenummer:
**PCT/EP98/03585**

(87) Internationale Veröffentlichungsnummer:
**WO 98/58325 (23.12.1998 Gazette 1998/51)**

(54) **VORRICHTUNG ZUM MESSEN UND ANALYSIEREN DER HIRNSTRÖME DER BEIDEN HIRNHÄLFTEN EINES PATIENTEN**

DEVICE FOR MEASURING AND ANALYZING CEREBRAL ELECTRICAL ACTIVITY IN BOTH CEREBRAL HEMISPHERES OF A PATIENT

DISPOSITIF POUR MESURER ET ANALYSER LES COURANTS CEREBRAUX DES DEUX HEMISPHERES CEREBRAUX D'UN PATIENT

(84) Benannte Vertragsstaaten:
**AT BE CH CY DE DK ES FI FR GB GR IE IT LI LU MC NL PT SE**

(30) Priorität: **15.06.1997 DE 19725214**

(43) Veröffentlichungstag der Anmeldung:
**05.04.2000 Patentblatt 2000/14**

(73) Patentinhaber:
• **Siebler, Mario**
  **42109 Wuppertal (DE)**
• **Theiss, Stephan**
  **40219 Düsseldorf (DE)**

(72) Erfinder:
• **Siebler, Mario**
  **42109 Wuppertal (DE)**
• **Theiss, Stephan**
  **40219 Düsseldorf (DE)**

(74) Vertreter: **Lenzing, Andreas, Dr.**
**Lenzing Gerber**
**Patentanwälte**
**Münsterstrasse 248**
**40470 Düsseldorf (DE)**

(56) Entgegenhaltungen:
**US-A- 4 339 810         US-A- 5 230 346**
**US-A- 5 357 976         US-A- 5 458 117**

• **JESSA M: "Chaotic numbers" COMMUNICATIONS ON THE MOVE. SINGAPORE. ICCS/ISITA '92(CAT. NO.92TH0479-6), SINGAPORE, 16-20 NOV. 1992, Seiten 50-52 vol.1, XP002081044 ISBN 0-7803-0803-4, 1990, New York, NY, USA, IEEE, USA**

**Beschreibung**

Die Erfindung betrifft ein Verfahren und eine Vorrichtung zum Messen elektrischer Hirnströme, und insbesondere auch zum Auswerten der Meßergebnisse.

In der Medizin stellt sich das Problem, die Funktionsfähigkeit eines menschlichen Gehirns nach dem Auftreten von beispielsweise einem Schlaganfall in der Akutphase zu messen.

Die elektrische Hirnaktivität kann als Elektroenzephalogramm (EEG) über empfindliche Verstärker von der Kopfhaut des Menschen gemessen werden, was 1910 von Berger entdeckt wurde. Schädigungen des Gehirns, wie z.B. Durchblutungsstörungen, äußern sich oft in Veränderungen des EEGs, d.h. Änderungen der Frequenzen oder Axnplituden der Signale. Dabei ist die Ortsspezifität, d.h. die exakte örtliche Auflösung des Schadensortes im Gehirn, gegenüber bildgebenden Verfahren, wie z.B. dem der Computertomographie des Schädels, sehr beschränkt. Hingegen ist die zeitliche Auflösung von Hirnaktivitäten oder Aktivitätsänderungen wesentlich besser. Zudem zeigt sich das volle Ausmaß der Hirnschädigungen in den Tomographien erst nach Stunden oder Tagen, wenn das Gewebe irreparabel zerstört ist.

Ein Messen eines EEG ist beispielsweise in der US-A-5,392,788 und der US-A-5,269,315 gezeigt.

Dabei dient das in der US-A-5,392,788 gezeigte System zum Prüfen der Reaktion eines Patienten auf Reize. Dabei werden Daten nach Erhalt verarbeitet, damit sie mit Grundwerten verglichen werden können. Abweichungen zwischen Daten und Grundwerten dienen zum Feststellen von Unregelmäßigkeiten.

Die US-A-S,269,315 zeigt ein Verfahren und eine Vorrichtung zum Interpretieren von Hirnströmen, wobei gleichzeitig in einem ersten Frequenzbereich und in einem zweiten Frequenzbereich bestimmte Hirnströme kombiniert werden. Nach einem Anwenden einer Fouriertransformation auf die digitalisierten gemessenen Daten erfolgt zur weiteren Verarbeitung der Daten eine Aufteilung der durch die Fouriertransformation erhaltenen Leistungsspektren in eine absolute Leistung und in eine relative Leistung.

Die US-A-5,458,117 zeigt ein System und ein Verfahren zum Ableiten eines Diagnoseindex aus 19 gemessenen EEG-Signalen. Bei der Auswertung der Signale wird auf alle gemessenen Signale beispielsweise eine schnelle Fouriertransformation oder eine Kreuzkorrelation angewendet. Die Ermittlung des Diagnoseindex ist schon aufgrund der vielen zu messenden Signale relativ aufwendig.

Somit sind zur objektiven Feststellung des Ausmaßes einer Hirnschädigung in der Akutphase bislang weder rein klinische Mittel noch bildgebende Verfahren, wie beispielsweise die Kernspintomographie, ausreichend, da sich bereits die Frage, ob überhaupt eine Durchblutungsstörung Ursache der beim Patienten vorliegenden Symptomatik ist, nicht auf einfache Weise beantworten läßt. Für bildgebende Verfahren ist ein hoher apparativer Aufwand erforderlich und sie lassen in der Akutphase, d.h. wenige Stunden nach dem Auftreten der Symptome, noch keine detaillierte Aussage über das Ausmaß der Schädigung zu. Da es aber insbesondere in der Akutphase wichtig ist, daß die richtigen Therapieentscheidungen getroffen werden, besteht ein großes Interesse an schnellen und einfachen Diagnosemethoden.

Die DE-OS 19 61 593 beschreibt die Durchführung eines Seitenvergleichs der EEG-Aktivität. Dabei ist die Positionierung der Elektroden ähnlich wie bei der vorliegenden Erfindung gewählt. Ein Wechselbeziehungskoeffizient gibt zu einer bestimmten Zeit an, ob die beiden untersuchten Kanäle die gleiche Polarität aufweisen. Werden darüber hinaus noch die Amplituden der Signale der Analyse hinzugefügt, wie es in der DE-OS 19 61 593 mittels des "Pearson-Produkt-Moment-Korrelationskoeffizienten" angedeutet ist (s. 5. 17), wird der Wert der Korrelationsfunktion der beiden Kanäle zu einer festen Zeitverschiebung At (hier bis zu 100 Bit bei 1 kHz = 0,1 sec.) bestimmt. Der Wechselbeziehungskoeffizient zeigt das instantane Verhalten der Polarität an; es wird kein zeitlicher Verlauf berechnet oder eine zeitliche Mittelung durchgeführt. Desweiteren werden bei dem in der DE-OS 19 61 593 beschriebenen Verfahren vorrangig sogenannte "evozierte Potentiale" gemessen, was bedeutet, daß der Patient einem (meist visuellen) Reiz ausgesetzt wird, woraufhin die elektrische kortikale Reaktion gemessen wird.

Die US 4 412 547 beschreibt eine Vorrichtung zum Überwachen von Hirnströmen, wobei das Meßverfahren mit wenig EEG- Elektroden auskommt. Bei der daruffolgenden Analyse der gemessenen Signale werden jedoch nur sehr grobe Eigenschaften des EEG-Signals verglichen: Die Leistung der einzelnen Kanäle (ggf. nach einer Frequenzfilterung) und die nach einem für ein so komplexes Signal fragwürdigen Verfahren ("zero crossing") bestimmte "mittlere Frequenz". Bei dem Verfahren der US 4 412 547 erfolgt die Umwandlung eines komplexen Frequenzgemischs in eine einzige Frequenzanzeige.

Ausgehend von der aus der US S 269 315 bekannten Vorrichtung liegt der Erfindung die Aufgabe zugrunde, eine Vorrichtung zu schaffen, mittels der ein Arzt bei der Untersuchung eines wahrscheinlich von einem Schlaganfall betroffenen Menschen schnell ein Entscheidungskriterium erhält, das eine möglichst sichere Prognose des Ausmaßes der Gehirnschädigung und ihrer zukünftigen Entwicklung erlaubt.

Diese Aufgabe wird durch die Vorrichtung des Patentanspruchs 1 gelöst. Die abhängigen Ansprüche 2 bis 9 zeigen vorteilhafte Weiterbildungen der Vorrichtung des Patentanspruchs 1.

Erfindungsgemäß werden sehr detaillierte Eigenschaften der gemessenen elektrischen Signale verwendet.

So wird eine Mustera- nalyse der FFT der Einzelkanäle und der FFT der Korrelationsfunktion auf ihre Peakstruktur hin (Lage, Höhe und Breite) durchgeführt. Die dann berechneten Struktureigenschaften werden verglichen, um daraus schließlich eine abgeleitete Größe, nämlich den Hirnfunktionsindex, zu bestimmen, die einer einfachen Interpretation zugänglich ist. Insbesondere erfolgt gemäß der Erfindung eine Analyse der Frequenzstruktur der gesamten Korrelationsfunktion.

Durch das erfindungsgemäße Verfahren zum Messen von elektrischen Hirnsignalen insbesondere eines Menschen kann insbesondere eine schnelle und effektive Analyse der gemessenen Signale erfolgen, die digitalisiert, gespeichert und nachfolgend basierend auf einem Computer analysiert werden.

Die erfindungsgemäße Vorrichtung ermöglicht eine schrittweise Verarbeitung der am Patienten gemessenen Hirnströme, wobei es insbesondere vorteilhaft ist, daß das Verfahren in Zusammenhang mit anderen klinischen Diagnoseverfahren durchgeführt werden kann.

Durch die Erfindung ist eine einfache, eindeutige Interpretation der Resultate sowie eine leichte Bedienbarkeit, wie beispielsweise durch Pflegepersonal, möglich. Weiterhin ist die erreichte Fehlerfestigkeit von großem Vorteil.

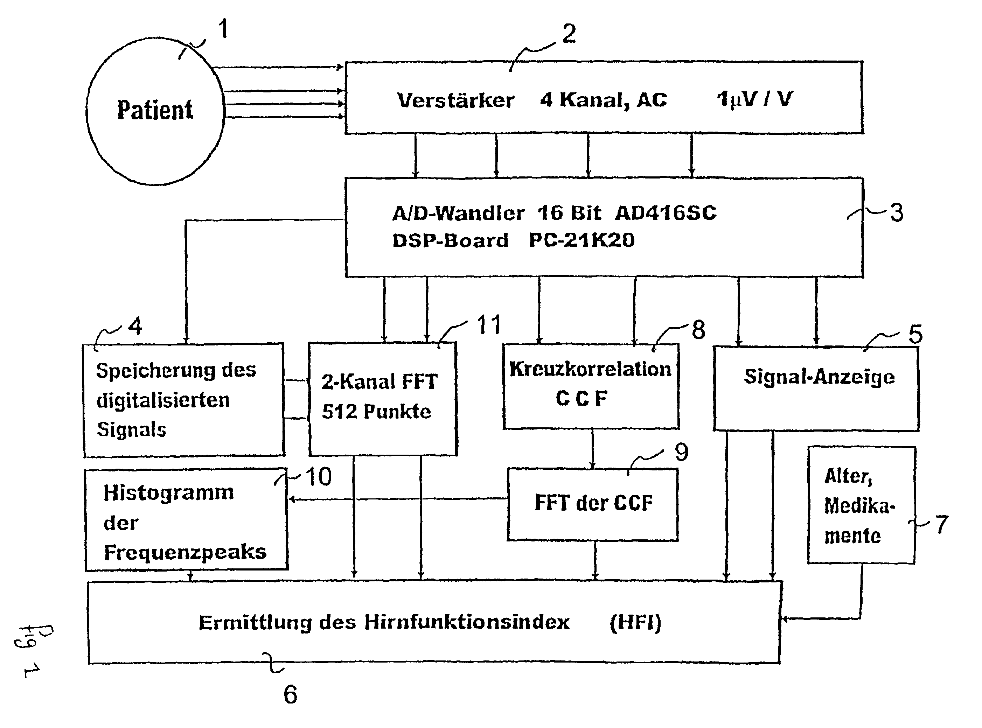

Im folgenden wird die Erfindung anhand eines Ausführungsbeispiels unter Bezugnahme auf die Figur detaillierter beschrieben.

Die Figur zeigt ein Blockschaltbild einer beispielhaften erfindungsgemäßen Vorrichtung zum Messen von Hirnströmen eines Patienten.

Am Kopf eines Patienten erfolgt die Signalaufnahme 1. Dabei werden an fünf Stellen des Patientenkopfes Meßelektroden angelegt: je ein Paar von Meßelektroden an jeder Seite des Kopfes, sowie eine zusätzliche Elektrode zur Erdung auf der Stirn. Aus den gemessenen Signalen werden die elektrischen Hirnsignale (EEG) abgeleitet und den Eingängen eines DC/AC-Verstärkers 2 zugeführt. Die EEG-Spannungen liegen im Bereich von μV, was eine gute Abschirmung der Zuleitungen erfordert. Die Abschirmung erfolgt am besten durch eine sogenannte "aktive Abschirmung".

Der beim vorliegenden Ausführungsbeispiel der Erfindung verwendete Verstärker hat eine Empfindlichkeit von 1 bis 5000 JLV/V, was einer Verstärkung von 200 bis 1000000 entspricht. Er ist über eine serielle Schnittstelle eines Notebooks/Pcs in bezug auf die Auswahl der Kanäle, die Festlegung der Meßbereicher die zuschaltung von Hoch- und Tiefpaßfiltern mit unterschiedlichen Grenzfrequenzen und die Impedanzmessung konfigurierbar und kann bei Bedarf auf bis zu acht Kanäle erweitert werden. Zum Unterdrücken unerwünschter Frequenzen im Signal werden schon im Verstärker ein Tiefpaßfilter mit einer oberen Grenzfrequenz von 30 Hz und zum Abschneiden von Gleichspannungsanteilen ein Hochpaßfilter mit einer unteren Grenzfrequenz von 0,2 Hz zugeschaltet. Eine automatische regelmäßige Impedanzmessung erfolgt mit einem Strom 1 = 5 V / 4,7 MΩ = 1,06 μA bei einer Meßfrequenz von 100 Hz (beim vorliegenden Ausführungsbeispiel einmal in 5 Minuten) und sichert, beispielsweise mit einem zusätzlichen akustischen Warnsignal, die Messung gegen unbemerktes Lösen der Elektroden, was beispielsweise aufgrund einer Bewegung des Patienten erfolgen kann. Eine automatische Anpassung des Meßbereichs des Verstärkers verhindert das "Clipping" bei eventuellem Ubersteuern des Verstärkers. Entscheidend für den Schutz vor Fehlmessungen ist dabei die Sicherstellung einer guten Signalqualität bei der Aufnahme, wofür das Anbringen der Elektroden am Kopf, die Impedanz des Übergangs von der Elektrode zum Kopf und die Einstellung des Meßbereichs des Verstärkers wichtig ist. Einem weiteren Schutz vor Fehlmessungen dient eine kontinuierliche Anzeige des Signals. Die Testmessungen erfolgten bei Übergangswiderständen von ca. 1 - 12 kΩ. Der Verstärkungsfaktor betrug etwa 200000.

Als nächstes erfolgt die Signalverarbeitung. Diese gesamte Verarbeitung der am Ausgang des Verstärkers 2 zur Verfügung stehenden Signale, die im Bereich von 1V liegen, erfolgt innerhalb eines PC oder eines Notebooks 3 mit einer Docking-Station. Dabei werden zwei spezielle Zusatzkarten für den PC verwendet. Von diesen Karten ist die eine eine Meßwerterfassungskarte (beim vorliegenden Ausführungsbeispiel ein MWE-Board mit der Typenbezeichnung AD416SC der Firma STAC GmbH) und die andere eine Digitalsignalprozessorkarte (beim vorliegenden Ausführungsbeispiel ein DSP-Board mit der Typenbezeichnung PC-21K20 der Firma STAC GmbH) zur numerischen Weiterverarbeitung der erfaßten und digitalisierten Signale.

Die Meßwerterfasssungskarte verfügt über vier differentielle Eingänge, die für Spannungen bis ± 10 V ausgelegt sind, frei wählbar DC- oder AC-gekoppelt werden können und je einen eigenen "Sample-and-Hold" -Meßverstärker sowie je einen 16-Bit -A/D-Wandler mit einer Dynamik von 90 dB enthalten. Besonderer Wert wird dabei auf optimal angepaßte Anti-Aliasing-Filter gelegt. Die Abtastung des Analogsignals erfolgt mit dem 512-fachen der gewünschten Zielabtastrate von 160 Hz, d.h. mit 81,92 kHz. Dafür besitzt jeder Kanal ein analoges und "weich" ausgelegtes ButterworthFilter, das praktisch keine Signalbeeinflussung im Meßbereich verursacht. Nach der A/D-Wandlung wird dann über scharfe, linearphasige Digitalfilter die erforderliche Bandbegrenzung für die Zielabtastrate sowie die Reduktion der Abtastrate um den Faktor 512 vorgenommen.

Die verwendeten Filter haben insbesondere folgende Eigenschaften:

- Durchlaßbereich (-3 dB) bei 48% der Zielabtastrate
- Ende des Durchlaßbereichs (-0,01 dB) bei 44% der Zielabtastrate

- Anfang des Sperrbereichs (-80 dB) bei 56% der Zielabtastrate.

Eine externe Referenzspannungsquelle für alle vier Wandler führt zu einer hohen Pegelgenauigkeit mit Schwankungen von weniger als 0,1%. Zwei Digitalsignalprozessorkarten und ein V40 Mikroprozessor, sowie Speicher in Form von 1 MB RAM auf der Karte entlasten den PC und übernehmen die Kommunikation mit der Digitalsignalprozessorkarte.

Die Digitalsignalprozessorkarte nutzt den 32-Bit-Gleitkommaprozessor ADSP 21020, der bis zu 60 MFLOPS leistet. Damit kann er beispielsweise eine schnelle Fouriertransformation von 1024 Punkten auf vier Kanälen bis zu einer Datenrate von 20 kHz in Echtzeit berechnen. Der im Vergleich zu den hier, auftretenden Anforderungen massive Überschuß an Rechenleistung wird dazu genutzt, den PC zu entlasten, mit sich überlappenden Zeitfenstern zu arbeiten, Mittelungen durchzuführen und Graphiken auf zubereiten. Die 32-Bit-Breite des Prozessors sichert den von der Meßwertermittlungskarte bereits vorgegebenen hohen Signal-Rausch-Abstand, der beim vorliegenden Ausführungsbeispiel bei der schnellen Fouriertransformation (=FFT) größer als 140 dB ist. Die an der Ausgangsseite der Digitalsignalprozessorkarte vorliegenden Daten haben dann eine Breite von 16 Bit und ganzzahlige Werte im Bereich von $\pm$ 32767. Die Zieldatenrate von 160 Hz ist so gewählt, daß die maximal meßbare Frequenz bei der Durchführung der FFT bei 80 Hz liegt und damit ein weiterer Abstand mit dem Faktor 4 zur höchsten bei der Auswertung berücksichtigten Frequenz vorhanden ist.

Als nächstes erfolgt eine Speicherung der Signale auf der Festplatte 4 des PC, der mit den Karten ausgerüstet ist. Ein Prototyp aus einem Notebook mit Docking-Station hat sich dabei wegen seiner leichten Handhabbarkeit am Krankenbett bewährt. Es kann auf dem Bett oder auf einer am Bett montierten Halterung verbleiben. Dabei entsteht kein unnötiger Kabelstrang zu einem im Raum stationären "Rechnerturm", der das Betreuen des Patienten erschwert. Zudem kann die Vorrichtung gemäß dem vorliegenden Ausführungsbeispiel auch an ein vorhandenes System zur Überwachung eines Patienten in einer sogenannten Stroke-Unit (Schlaganfall-Intensiveinheit) angepaßt werden, mit welchem zusätzlich noch Messungen des Elektrokardiogramms, des Blutdrucks, der Sauerstoffsättigung usw. vorgenommen werden. Beim vorliegenden Ausführungsbeispiel wurde zur Signalspeicherung das Recorder-Programm der Firma STAC GmbH verwendet, das optimal auf die Karten derselben Firma abgestimmt ist. Es erlaubt eine Online-Speicherung der zwei differenzverstärkten EEG-Kanäle (jeweils central/temporal gegen Mastoid) auf einer handelsüblichen Festplatte. Dabei entstehen Dateien in der Größenordnung von 10 min * 160 Hz * 2 Kanäle * 2 Byte 384000 Bytes pro Ableitung. Während der Aufnahme kann das Signal selbst sowie beispielsweise auch sein Spektrum auf dem Monitor 5 des Notebooks angezeigt werden.

Nach einer abgeschlossenen Messung der Signale erfolgt die eigentliche Analyse 8, 9, 10, 11 der Signale aus den in der Datei gesicherten Daten. Das bedeutet, daß es zum einen keinen Nachteil gegenüber einer direkten Online-Analyse gibt, weil die Meßdauer mit 5-10 Minuten hinreichend kurz ist und die nachfolgende Analyse in etwa der Hälfte der Echtzeit durchgeführt werden kann, und daß es zum anderen den Vorteil gibt, daß sie jederzeit, beispielsweise mit geänderten Parametern, wiederholt werden kann. Auf einem hinreichend leistungsfähigen System ist es denkbar, daß die Analyse direkt parallel zur Signalspeicherung durchgeführt werden kann.

Die Analyse eines diskreten, aber nicht zeitbegrenzten Signals durch eine diskrete Fouriertransformation (=DFT) erfordert immer die Aufteilung in Blöcke. Dabei ist zu beachten, daß durch die Blocklänge und die Abtastrate ebenfalls die Frequenzauflösung, d.h. die niedrigste meßbare Frequenz, und die maximale meßbare Frequenz festgelegt sind. Zwischen diesen gelten die Beziehungen

Frequenzauflösung $\Delta f = f_s/N$ Zeitsignal-Blocklänge $T = N^*\Delta t = N/f_s$

Zeitauflösung $\Delta t = l/f_s$ Abtastrate $f_s = l/\Delta t$

Insbesondere ist es wichtig, daß das Signal vor der Digitalisierung keine Frequenzanteile oberhalb der halben Sampling- oder Abtastrate enthält, die sich durch Aliasing-Effekte bemerkbar machen würden und die Effekte stark verfälschen könnten (Abtast-Theorem). Die Anpassung der Anti-Aliasing-Filter erfolgt schon hardwareseitig durch die Meßwertermittlungskarte bei softwareseitig (STAC-Recorder) eingestellter Abtastrate.

Die Korrelationsfunktion zweier quadratintegrabler Funktionen f1 und f2 ist mathematisch definiert als

$$C(\tau) = \int f_1(t) f_2(t+\tau) \, dt = \sum_{t=1}^{N} f_1(t) f_2(t+\tau),$$

Für zeitlich unbegrenzte Signale die in der Regel nicht quadratintegrabel sind kann sie daher nicht berechnet werden. Zerlegt man die Signale f1 und f2 in - sich gegebenenfalls - überlappende Blöcke ("Zeitfenster"), kann man allerdings die Korrelation zweier Funktionen berechnen, die innerhalb des Zeitfensters durch das Signal gegeben und außerhalb gleich Null sind. Die hier vorliegenden "Funktionen" sind natürlich nicht kontinuierlich, sondern durch das Abtasten bzw. Sampling diskret definiert, so daß an die Stelle des Integrals die Summendarstellung tritt. Diese Berechnung der Kreuzkorrelation liefert dann eine diskrete Funktion, die auf einem Intervall der doppelten Länge T des Originalsignals definiert und außerhalb des Intervalls [-T, T] gleich Null ist.

Oft wird bei ähnlichen Anwendungen die Korrelationsfunktion mittels inverser Fouriertransformation aus der FFT der Einzelsignale berechnet, was vom numerischen Aufwand her Vorteile hat. Allerdings setzt die DFT das Zeitsignal nach einer Blocklänge exakt periodisch fort, was dann in der Regel zu einem unstetigen Signalverlauf führt. Um Diskontinuitätseffekte zu vermeiden, multipliziert man normalerweise das Signal vor der Transformation mit einer Fensterfunktion, wie beispielsweise dem HanningFenster, die das Signal an den Rändern des Blocks differenzierbar auf Null reduziert, und arbeitet dafür mit einer überlappung der Zeitfenster von in der Regel mehr als 60%. Das ist aber bei der Berechnung der Kreuzkorrelationsfunktion (=CCF) nicht angebracht, da eine Fensterfunktion das Signal zu sehr beeinflussen und stark in die berechnete CCF eingehen würde. Aus diesem Grund entfällt auch die Notwendigkeit, das Signal mit einem großen "Overlap" oder großer Überlappung abzutasten, da am Rand zweier Blöcke keine Signalinformation gedämpft wird. Gemäß dem vorliegenden Ausführungsbeispiel wird die Kreuzkorrelation daher direkt gemäß der obenstehenden Summenformel berechnet.

Die berechnete Korrelation 8 beschreibt, inwieweit das Signal des einen Kanals dem des anderen Kanals ähnlich ist, wenn es um eine bestimmte Zeit verschoben wird. Sie kann somit eine Kohärenz zwischen den beiden Hirnhälften detektieren. Um nun herauszufinden, ob in beiden Hirnhälften synchron dieselbe Grundfrequenz vorhanden ist, wird die Korrelationsfunktion selbst auch einer schnellen Fouriertransformation oder FFT 9 unterzogen. Dabei wird die Abtastrate halbiert, d.h. die Frequenzauflösung wird verdoppelt. Aus dem Spektrum der Kreuzkorrelationsfunktion oder CCF wird der Bereich von 0-20 Hz angezeigt, der dann 128 Punkte umfaßt. Das Augenmerk gilt dann dem Vorhandensein eines oder mehrerer Spitzenwerte oder "Peaks" im CCF-Spektrum im Bereich von 3 bis 14 Hz, vorzugsweise von 7 bis 12 Hz. Da dieser Peak auch beim gesunden Menschen nicht permanent vorhanden sein muß, wird ein zeitlicher Mittelwert über einen Zeitraum von einer bis zehn Minuten in Form eines Histogramms 10 gebildet.

Bei der mit dem vorliegenden Ausführungsbeispiel durchgeführten Testreihe hat sich die Blocklänge von 512 Werten bewährt. Das entspricht bei einer Samplingrate von 160 Hz einer Zeitfenstergröße von (T = 512 / 160 Hz=) 3,2 Sekunden und einer Frequenzauflösung von ($\Delta$f = 1 / 3,2 sek.=) 0,31 Hz. Damit entfallen auf den für die spätere Analyse interessanten Frequenzbereich von 0-20 Hz 64 Punkte. Zu Testzwecken wurde mit einer überlappung von 60% gearbeitet, was "neuen" Werten, in 1,28 sek. pro Block mit 3,2 sek. entspricht. In der Praxis empfiehlt sich eine geringe Uberlappung von etwa 10%.

Bei der Diagnoseerstellung wurde von der Hypothese ausgegangen, daß sich das Ausmaß der Schädigung des Gehirns durch die Durchblutungsstörung besonders darin niederschlägt, wie stark die beiden Hirnhälften noch in der Lage sind, $\alpha$-Frequenzen zu erzeugen, sich zu "synchronisieren" und "in Phase" zu arbeiten. Diese Synchronisation wird beim gesunden Menschen durch einen Frequenzanteil im Bereich von 3 bis 14 Hz und vorzugsweise im Bereich von 7 bis 12 Hz hergestellt, der $\alpha$-Bereich oder "Grundrhythmus" genannt wird. Dafür ist zum einen notwendig, daß ein solcher Frequenzanteil in beiden Hirnhälften gemessen wird, also auf beiden Kanälen gemessen werden kann, und zum anderen, daß dieser Anteil in beiden Hälften bei derselben Frequenz liegt. Deshalb werden gemäß der Erfindung nicht nur die Spektren der Einzelsignale 11 untersucht, sondern es wird auch eine Funktion betrachtet, die "Ähnlichkeiten" der beiden Kanäle in Form der Kreuzkorrelationsfunktion 8 mißt. Nur wenn der Grundrhythmus auf beiden Seiten bei derselben Frequenz liegt, wird sich eine entsprechende Frequenzkomponente auch in der Kreuzkorrelation zeigen. Als Ergebnis wird ein sogenannter Hirnfunktionsindex (HFI) 6 zu einem Maß für die Normalität der Hirnfunktion des Untersuchten berechnet. Er kann die drei Werte "normal", "leicht betroffen" und "pathologisch" annehmen. Seine Ermittlung kann sich aber nicht auf die Signalanalyse allein stützen. Es ist beispielsweise bekannt, daß junge Menschen unter 20 Jahren noch keinen ausgeprägten $\alpha$-Rhythmus zeigen und daß das EEG von Menschen, die unter dem Einfluß von Medikamenten (Beruhigungs-, Schmerzmittel, Valium) stehen, stark modifiziert sein kann.

Deshalb gehen klinische Parameter 7 in die Berechnung des Hirnfunktionsindex ein. Solche Parameter 7 sind beispielsweise Alter des Patienten oder Medikamenteneinfluß. Der Hirnfunktionsindex (HFI) wird also berechnet aus:

- Alter und Medikamenteneinfluß;
- Signalqualität der mit der Vorrichtung zum Messen von Hirnströmen ermittelten Parameter;
- Frequenzpeak zwischen 3 und 14 Hz, vorzugsweise zwischen 7 und 12 Hz in beiden Kanälen der FFT des EEG-Signals;
- Frequenzpeak zwischen 3 und 14 Hz, vorzugsweise zwischen 7 und 12 Hz in der FFT der Kreuzkorrelationsfunktion;
- Scharfe Verteilung im Histogramm der Frequenzen der Peaks in der FFT der Kreuzkorrelation; und
- Anzahl der simultan auftretenden Frequenzpeaks im $\alpha$-Bereich.

Bei guter Signalqualität, einem Patienten von über 20 Jahren, der nicht unter dem Einfluß von Beruhigungsmitteln steht, erfolgt damit dann folgende Zuordnung:

Zeigt die FFT eines EEGs auf einer Seite keinen Peak im o.a. Bereich, und zeigen auch die Korrelationsfunktion und das Histogramm keinen solchen Peak, so gilt HFI = pathologisch.

Zeigt die FFT eines EEGs auf beiden Seiten einen Peak im o.a. Bereich, und zeigen auch die Korrelationsfunktion und das Histogramm einen solchen Peak, so gilt HFI = normal.

Zeigt die FFT eines EEGs auf einer Seite gelegentlich einen Peak im oben angegebenen Bereich, und zeigt die Korrelationsfunktion gelegentlich keinen Peak im oben angegebenen Bereich, und zeigt das Histogramm (als Mittel) einen solchen Peak (wenn auch schwächer ausgeprägt), so gilt HFI = leicht betroffen.

Durch die Erfindung ist es also für einen Anwender möglich, wichtige und aussagekräftige Zusatzinformationen zur Diagnose bei einem Patienten mit Schlaganfall in der Akutphase zu erhalten. Die Auswertung erfolgt nach einer Speicherung gemessener Daten schnell und kann durch die Speicherung beliebig oft wiederholt werden. Zur Auswertung werden nicht nur die gemessenen Hirnsignale herangezogen, sondern die Auswertung dieser gemessenen Werte erfolgt auch unter Zuhilfenahme von Zusatzinformation über den Patienten, die als Parameter eingegeben werden. Als Ergebnis der Auswertung durch die Vorrichtung der Erfindung mit dem Verfahren gemäß der Erfindung wird ein kurze Mitteilung über die Schwere der Schädigung in Form eines sogenannten Hirnfunktionsindex ausgegeben.

## Patentansprüche

1. Vorrichtung zum Messen und Analysieren der Hirnströme der beiden Hirnhälften eines Patienten mit

   einer Einrichtung (1) zum Messen von Hirnströmen in den zwei Hirnhälften eines Patienten in Form von elektrischen Signalen durch Anlegen von wenigen Meßelektroden am Kopf des Patienten;
   einem Verstärker (2) zum Verstärken der gemessenen Signale; und
   einem Computer mit Zusatzmitteln, wie
   einem A/DWandler (3) zum Umwandeln der verstärkten analogen Signale in digitalisierte Signale,
   einer Einrichtung (8) zur Berechnung der Kreuzkorrelationsfunktion (CCF) aus den digitalisierten Signalen der beiden einzelnen Kanäle, um festzustellen, inwieweit sich die gemessenen Signale zweier Kanäle gleichen, was ein Maß für die Kohärenz der elektrischen Aktivität zwischen den beiden Hirnhälften eines Patienten ist, wodurch die Kreuzkorrelationsfunktion (CCF) erhalten wird,
   einem Speicher (4) zum Speichern der digitalisierten Signale,
   einer Einrichtung (11) zum Anwenden einer schnellen Fouriertransformation in einem bestimmten Frequenzbereich auf die digitalisierten und/oder die gespeicherten digitalisierten Signale der beiden einzelnen Kanäle, um die Struktur der Spektren der Einzelsignale zu berechnen und vergleichen,
   einer Einrichtung (9) zum Anwenden einer schnellen Fouriertransformation (FFT) auf die Kreuzkorrelationsfunktion in einem bestimmten Frequenzbereich, um die Spektren der Kreuzkorrelationsfunktion zu berechnen und analysieren, mit dem Ziel, festzustellen, ob in beiden Hirnhälften synchron dieselben Grundfrequenzen vorhanden sind, und einer Einrichtung (6) zur Ermittlung eines Hirnfunktionsindex als Maß für die Normalität der Hirnfunktion des Patienten, der durch Verwendung sowohl der einer schnellen Fouriertransformation (FFT) unterzogenen Kreuzkorrelationsfunktion als auch der einer schnellen Fouriertransformation (FFT) unterzogenen Signale der beiden einzelnen Kanäle ermittelt wird.

2. Vorrichtung nach Anspruch 1, **dadurch gekennzeichnet,** daß die Einrichtung (8) zur Berechnung der Kreuzkorrelationsfunktion (CCF) für die digitalisierten Signale der beiden einzelnen Kanäle die digitalisierten Signale vor der Berechnung der Kreuzkorrelationsfunktion (CCF) in einem Fensterbereich in sich überlappende Blöcke aufteilt.

3. Vorrichtung nach Anspruch 1 oder 2, **dadurch gekennzeichnet,** daß die Einrichtung (11) zum Anwenden einer schnellen Fouriertransformation (FFT) auf die digitalisierten und/oder die gespeicherten digitalisierten Signale der beiden einzelnen Kanäle die Signale vor dem Anwenden der schnellen Fouriertransformation (FFT) in sich überlappende Blöcke auf teilt

4. Vorrichtung nach einem der vorangehenden Ansprüche, **dadurch gekennzeichnet,** daß die Zusatzmittel des Computers weiterhin eine Einrichtung (10) zum Bilden eines Histogramms von Frequenzpeaks aus der der schnellen Fouriertransformation (FFT) unterworfenen Kreuzkorrelationsfunktion in einem bestimmten Frequenzbereich zum Ermitteln einer Amplituden-Verteilung in diesem Bereich aufweisen, wobei durch die Analyse der Struktur des gesamten Signals, das sich aus den Signalen beider Kanäle und der Kreuzkorrelationsfunktion (CCF) zusammensetzt, für jeweilige Blöcke im Frequenzbereich Kennzahlen berechnet werden, die dann ihrerseits zeitlich über eine bestimmte Zeit gemittelt werden, und aus deren Mittelung der Hirnfunktionsindex berechnet wird.

5. Vorrichtung nach einem der vorangehenden Ansprüche, **dadurch gekennzeichnet,** daß sie weiterhin Anzeigevorrichtungen (5, 6) zum Anzeigen von Meßsignalen und/oder des ermittelten Hirnfunktionsindex aufweist, wobei die digitalisierten Signale während der Signalerfassung auf den Anzeigevorrichtungen anzeigbar sind.

6. Vorrichtung nach Anspruch 5, **dadurch gekennzeichnet**, daß die Anzeigevorrichtungen (5, 6) den ermittelten Hirnfunktionsindex in Wort und/oder Bild als Diagnoseergebnis in Form von beispielsweise "HFI=pathologisch", "HFI=normal" oder "HFI=leicht betroffen" oder als Index bzw. Zahlenwert kurz und allgemein verständlich anzeigen.

7. Vorrichtung nach einem der vorangehenden Ansprüche, **dadurch gekennzeichnet**, daß sie weiterhin eine Eingabeeinrichtung (7) aufweist, die zum Eingeben von bei der Ermittlung des Hirnfunktionsindex nützlichen zusätzlichen Parametern in die Einrichtung (6) zur Ermittlung des Hirnfunktionsindex dient, die beispielsweise Alter des Patienten und/oder Medikamenteneinnahme sein können.

8. Vorrichtung nach einem der vorangehenden Ansprüche, **dadurch gekennzeichnet,** daß sie weiterhin eine Abrufeinrichtung (7) aufweist, die zum Abrufen von bei der Ermittlung des Hirnfunktionsindex zuvor gespeicherten nützlichen zusätzlichen Parametern dient, die beispielsweise Alter des Patienten und/oder Medikamenteneinnahme sein können.

9. Vorrichtung nach einem der vorangehenden Ansprüche, **dadurch gekennzeichnet,** daß sie in ein bestehendes System zum Messen weiterer Werte für Diagnosezwecke integrierbar ist.

**Claims**

1. An apparatus for measuring and analyzing the brain currents of both brain hemispheres of a patient and having a device (1) for measurement of brain currents in the two brain hemispheres of a patient in the form of electrical signals by application of several measurement electrodes on the patient's head; an amplifier (2) for amplifying the measured signals; and a computer with accessories, such as an A/D converter (3) for conversion of the amplified analog signals into digital signals, a device (8) for calculation of the cross-correlation function (CCF) from the digitized signals of the two individual channels in order to ascertain the extent to which the measured signals from two channels are similar - this is a measurement of the coherence of the electrical activity between the two brain hemispheres of a patient - from which the cross-correlation function (CCF) is obtained; a memory (4) for storage of the digitized signals; a device (11) for application of a fast Fourier transformation in a given frequency range to the digitized signals and/or the stored digitized signals of the two individual channels for the purpose of calculating and comparing the structure of the spectra of the individual signals.
A device (9) for the purpose of applying a fast Fourier transformation (FFT) to the cross-correlation function in a certain frequency range in order to calculate and analyze the spectra of the cross-correlation function. The purpose of this is to ascertain whether in both brain hemispheres the same basic frequencies are synchronously present, and a device (6) for the determination of a brain function index as the measurement for the normalcy of the patient's brain function, which is determined by the use of both a cross-correlation function which has been subjected to a fast Fourier transformation (FFT) and the Signals of both individual channels subjected to a fast Fourier transformation (FFT).

2. A device as described in Claim 1, **characterized by the fact, that;** the device (8) separates the digitized signals, prior to calculation of the cross-correlation function (CCF) in a data range, into overlapping blocks for the purpose of calculating the cross-correlation function (CCF) of the two individual channels.

3. A device as described in Claim 1 or Claim 2, **characterized by the fact, that** the device (11) blocks, prior to the application of the fast Fourier transformation (FFT), separates the signals into overlapping blocks for the purpose of application of a fast Fourier transformation (FFT) on the digitized and/or the stored digitized signals of the two individual channels.

4. Advice as described in one of the foregoing claims and
**characterized by the fact, that** the supplement utilities of the computer further includes a device (10) for the creation of a histogram of frequency peaks from which the cross-correlation functions subjected to the fast Fourier transformation (FFT) are shown in a particular frequency range for the purpose of determining an amplitude distribution in said range, and whereby by analysis of the structure of the entire signal that is composed of the signals of both channels and the cross-correlation function (CCF), characteristic numbers are calculated for current blocks in the frequency range, which are then temporally averaged over a given time period and from whose averaging the brain function index is calculated.

5. A device as described in the foregoing Claims and
**characterized by the fact, that** has additional display devices (5, 6) for displaying of measured signals and/or the derived brain function index, whereby the digitized signals can be displayed on the display devices during signal pickup.

6. Advice as described in Claim 5 and **characterized by the fact, that** the display devices (5, 6) display the determined brain function index in text and/or graphics as the diagnostic result in the form of, for example, "HFI=Pathology," "HFI=Normal," or "HFI=Mildly Affected" or as an index or numeric value that is concise and generally understandable.

7. Advice in accordance with one of the foregoing claims and
**characterized by the fact, that** it has an input device (7) which is used for entering useful additional parameters into the device (6) at the time of determining the brain function index and for the purpose of determining the brain function index and which, for example, may be the age of the patient and or information relating to medications being taken.

8. A device in accordance with one of the foregoing claims and
**characterized by the fact, that** it has a call-up device (7) which is used, at the time of determining the brain function index, for the purpose of calling up previously stored useful additional parameters that can be, for example, the age of the patient and/or information relating to medications being taken.

9. Advice in accordance with one of the foregoing claims and
**characterized by the fact, that** it can be integrated into an existing system for the purpose of measuring other values for the purpose of diagnosis.

**Revendications**

1. Dispositif pour mesurer et analyser les courants cérébraux des deux hémisphères cérébraux d'un patient, comportant

un dispositif (1) pour mesurer des courants cérébraux dans les deux hémisphères cérébraux d'un patient sous la forme de signaux électriques par application de quelques électrodes de mesure sur la tête du patient;
un amplificateur (2) pour amplifier les signaux mesurés; et
un ordinateur comportant des moyens additionnels, comme par exemple
un convertisseur analogique/numérique (3) pour convertir les signaux analogiques amplifiés en des signaux numérisés,
un dispositif (8) pour calculer la fonction de corrélation croisée (CCF) à partir des signaux numérisés des deux canaux individuels, pour déterminer dans quelle mesure les signaux mesurés de deux canaux sont identiques, ce qui est une mesure de la cohérence de l'activité électrique entre les deux hémisphères cérébraux d'un patient, ce qui permet d'obtenir la fonction de corrélation croisée (CCF),
une mémoire (4) pour mémoriser des signaux numérisés,
un dispositif (11) pour appliquer une transformation de Fourier rapide dans une gamme déterminée de fréquences aux signaux numérisés et/ou aux signaux numérisés mémorisés des deux canaux individuels, de manière à calculer et comparer la structure des spectres des signaux individuels,
un dispositif (9) pour appliquer une transformation de Fourier rapide (FFT) à la fonction de corrélation croisée dans une gamme déterminée de fréquences, pour calculer et analyser les spectres de la fonction de corrélation croisée, avec comme objectif de déterminer si les mêmes fréquences de base sont présentes d'une manière synchrone dans les deux hémisphères cérébraux, et un dispositif (6) pour déterminer un indice de fonctionnement cérébral en tant que mesure de la normalité de la fonction cérébrale du patient, qui est déterminée par utilisation aussi bien de la fonction de corrélation croisée soumise à une transformation de Fourier rapide (FFT), que des signaux, soumis à la transformation de Fourier rapide (FFT), des deux canaux individuels.

**2.** Dispositif selon la revendication 1, **caractérisé en ce que** le dispositif (8) servant à effectuer la fonction de corrélation croisée (CCF) pour les signaux numérisés des deux canaux individuels, divise des blocs, qui se chevauchent, des signaux numérisés avant le calcul de la fonction de corrélation croisée (CCF), dans une zone formant fenêtre.

**3.** Dispositif selon la revendication 1 ou 2, **caractérisé en ce que** le dispositif (11) servant à appliquer une transformation de Fourier rapide (FFT) aux signaux numérisés et/ou aux signaux numérisés et mémorisés des deux canaux individuels, subdivise les signaux en des blocs qui se chevauchent, avant l'application de la transformation de Fourier rapide (FFT).

**4.** Dispositif selon l'une des revendications précédentes, **caractérisé en ce que** les moyens supplémentaires de l'ordinateur comportent en outre un dispositif (10) pour former un histogramme de pics de fréquences à partir de la fonction de corrélation croisée soumise à la transformation de Fourier rapide (FFT), dans une gamme déterminée de fréquences pour déterminer une distribution d'amplitude dans cette gamme, auquel cas grâce à l'analyse de la structure dans l'ensemble du signal qui se compose ici des deux canaux et de la fonction de corrélation croisée (CCF), des nombres caractéristiques sont calculés pour des blocs respectifs dans la gamme des fréquences, nombres caractéristiques, qui pour leur part sont soumis à une formation de la moyenne dans le temps, et ce pendant un intervalle de temps déterminé, et l'indice de fonctionnement cérébral est calculé à partir de la formation de la moyenne de ces nombres caractéristiques.

**5.** Dispositif selon l'une des revendications précédentes, **caractérisé en ce qu'**il comporte en outre des dispositifs d'affichage (5,6) servant à afficher des signaux de mesure et/ou l'indice déterminé de fonctionnement cérébral, les signaux numérisés pouvant être affichés pendant la détection des signaux, sur les dispositifs d'affichage.

**6.** Dispositif selon la revendication 5, **caractérisé en ce que** les dispositifs d'affichage (5,6) affichent l'indice déterminé de fonctionnement cérébral d'une manière abrégée et d'une manière compensée d'une manière générale par un mot et/ou une image en tant que résultat de diagnostic sous la forme par exemple "HFI = pathologique", "HFI = normal" ou "HFI = léger", ou bien sous la forme d'un indice ou d'une valeur chiffrée.

**7.** Dispositif selon l'une des revendications précédentes, **caractérisé en ce qu'**il comporte en outre un dispositif d'entrée (7) qui pour l'entrée de paramètres supplémentaires utiles lors de la détermination de l'indice de la fonction cérébrale, dans le dispositif (6) sert à déterminer l'indice de la fonction cérébrale, ces paramètres pouvant être par exemple l'âge du patient et/ou la prise de médicament.

**8.** Dispositif selon l'une des revendications précédentes, **caractérisé en ce qu'**il comporte en outre un dispositif d'appel (7) qui sert à appeler des paramètres supplémentaires utiles préalablement mémorisés lors de la détermination de l'indice de fonctionnement cérébral et qui peuvent être par exemple l'âge du patient et/ou la prise d'un médicament.

**9.** Dispositif selon l'une des revendications précédentes, **caractérisé en ce qu'**il peut être intégré dans un système existant pour la mesure d'autres valeurs dans un but de diagnostic.

```
        ╭─ 1                    ╭─ 2
   ┌─────────┐        ┌──────────────────────────────────────┐
   │ Patient │───────>│ Verstärker   4 Kanal, AC    1µV / V   │
   └─────────┘        └──────────────────────────────────────┘
                                      │  │  │  │
                      ┌──────────────────────────────────────┐  ╭─ 3
                      │ A/D-Wandler  16 Bit  AD416SC          │
                      │ DSP-Board   PC-21K20                  │
                      └──────────────────────────────────────┘
```

| ╭─ 4 | ╭─ 11 | ╭─ 8 | ╭─ 5 |
|---|---|---|---|
| Speicherung des digitalisierten Signals | 2-Kanal FFT 512 Punkte | Kreuzkorrelation C C F | Signal-Anzeige |

| ~10 Histogramm der Frequenzpeaks | ╭─ 9 FFT der CCF | Alter, Medika-mente ~7 |
|---|---|---|

Ermittlung des Hirnfunktionsindex     (HFI)

6

fig 1